# EUROPEAN PATENT APPLICATION

(11) **EP 2 998 274 A1**
(43) Date of publication of application: **23.03.2016**
(21) Application number: 14798638.4
(22) Date of filing: 11.04.2014
(51) Int. Cl.: C01F 7/00, A61K 8/19, A61K 8/26, A61K 8/27, A61Q 1/12, C01G 9/00

(54) **LAYERED DOUBLE HYDROXIDE CAPABLE OF ADSORBING UNSATURATED FATTY ACIDS SELECTIVELY, AND COSMETIC PRODUCED USING SAID LAYERED DOUBLE HYDROXIDE**

(30) Priority: 13.05.2013 JP 2013101751
(71) Applicant: Tayca Corporation, Osaka-shi, Osaka 551-0022 (JP)
(72) Inventor: TAKADA Akihiko, Osaka-shi Osaka 551-0022 (JP); OKUMIYA Takeshi, Osaka-shi Osaka 551-0022 (JP); YAMASHITA Jun, Osaka-shi Osaka 551-0022 (JP)
(74) Representative: Cabinet Plasseraud
(86) International application number: PCT/JP2014/060478
(87) International publication number: WO 2014/185201

(57) **Abstract**

[Object] A layered double hydroxide which has selective adsorbability to unsaturated fatty acids and can have a neutral pH value (i.e., a weakly acidic to weakly alkaline pH value) upon being dispersed in water and a cosmetic produced using the layered double hydroxide have been demanded. [Solution] The layered double hydroxide according to the present invention is characterized by comprising base layers each comprising a metal double hydroxide represented by the formula: M(II)1-XM(III)X(OH)2 (wherein M(II) represents one or two bivalent metal atoms; M(III) represents a trivalent metal atom; and x represents 0.2 to 0.33), and an intermediate layer and interlayer water each intercalated between the base layers, wherein the intermediate layer comprises a compound represented by the formula: R1-COOH or R2-SO3H (wherein R1 and R2 independently represent at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group).

## Description

### Technical Field

The present invention relates to a layered double hydroxide, more specifically to a layered double hydroxide which has selective adsorbability to unsaturated fatty acids (in particular, oleic acid) and a cosmetic produced using this layered double hydroxide.

### Background Art

Layered double hydroxides (hydrotalcite-like compounds) are compounds represented by the general formula [M²⁺₁₋ₓM³⁺ₓ (OH)₂] [Aⁿ⁻_{x/n} · mH₂O] and are known as compounds which have an anion exchange capability. Specifically, such a compound has a structure including base layers formed of bivalent and trivalent metal hydroxides, and an intermediate layer and interlayer water each intercalated between the base layers.

Such layered double hydroxides can be formed so as to exhibit various characteristics depending on combinations of bivalent and trivalent metal atoms forming base layers and anions forming intermediate layers. Thus, various layered double hydroxides have been developed.

The applicant of the present application also developed layered double hydroxides in which bivalent and trivalent metal atoms are Mg and Al and various materials are intercalated (refer to Patent Literatures 1 to 3).

Also, the applicant of the present application found that use of Mg and Al as the bivalent and trivalent metal atoms and use of magnesium acetate or magnesium acrylate as the intercalation compound provide a technical advantage of selective adsorbability to unsaturated fatty acids such as oleic acid. The applicant applied for a patent directed to such a layered double hydroxide and obtained the patent (refer to Patent Literature 4).

It is known that such unsaturated fatty acids are contained in human sebum components or decomposed substances derived from sebum components, and cause makeup coming off or shine. On the other hand, sebum components also include skin-moisturizing components such as squalene.

Accordingly, layered double hydroxides which have selective adsorbability to the unsaturated fatty acids, which cause makeup coming off or shine, are highly useful in the cosmetic field. In particular, among unsaturated fatty acids, oleic acid accounts for about 30% to about 40% of sebum components. Thus, layered double hydroxides which have selective adsorbability to oleic acid are very highly useful in the cosmetic field.

### Citation List

### Patent Literature

PTL 1: Japanese Patent No. 5022038
PTL 2: Japanese Patent No. 5155568
PTL 3: Japanese Patent No. 5178027
PTL 4: Japanese Patent No. 5065777

### Summary of Invention

### Technical Problem

The layered double hydroxide described in PTL 4 has selective adsorbability to unsaturated fatty acids; however, it becomes strongly alkaline (pH: about 10) upon being dispersed in water. Thus, in consideration of, for example, the influence of the layered double hydroxide on the skin, this hydroxide itself is difficult to use as a cosmetic, which has been problematic.

In addition, the layered double hydroxide described in PTL 4, which employs magnesium acetate as the intercalation compound, has a problem of emission of acetic acid odor. This is also the reason why this hydroxide itself is difficult to use as a cosmetic, which has been problematic.

This time, the inventors of the present invention performed thorough studies. As a result, the inventors have found that use of a compound represented by a specific chemical formula as the intercalation anion can provide selective adsorbability to unsaturated fatty acids and can also allow a neutral pH value (i.e., a weakly acidic to weakly alkaline pH value) even upon being dispersed in water.

In view of the above-described known problems, the present invention has been made. An object is to provide a layered double hydroxide which has selective adsorbability to unsaturated fatty acids and can also have a neutral pH value (i.e., a weakly acidic to weakly alkaline pH value) even upon being dispersed in water, and a cosmetic produced using this layered double hydroxide.

### Solution to Problem

In order to achieve the above-described object, a layered double hydroxide according to the present invention is characterized by including base layers each including a metal double hydroxide represented by the formula: M(II)₁₋ₓM(III)ₓ(OH)₂ (wherein M(II) represents one or two bivalent metal atoms; M(III) represents a trivalent metal atom; and x represents 0.2 to 0.33), and an intermediate layer and interlayer water each intercalated between the base layers, wherein the intermediate layer is a compound represented by the following Formula 1 or Formula 2

[Formula 1] R₁-COOH

(wherein R₁ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group)

[Formula 2] R₂-SO₃H

(wherein R₂ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group).

A layered double hydroxide according to the present invention is characterized in that M(II) represents Zn and M(III) represents Al.

A layered double hydroxide according to the present invention is characterized in that M(II) represents Mg and Zn, and M(III) represents Al.

A layered double hydroxide according to the present invention is characterized in that the compound represented by Formula 1 is at least one compound selected from salicylic acid, hydroxybenzoic acid, aminobenzoic acid, methoxybenzoic acid, pentanoic acid, dodecanoic acid, octadecanoic acid, docosanoic acid, isopentanoic acid, isododecanoic acid, isooctadecanoic acid, 4-aminobutyric acid, 6-aminohexanoic acid, tranexamic acid, picolinic acid, taurine, pyrrolidonecarboxylic acid, and sodium N-lauroylsarcosinate.

A cosmetic according to the present invention is characterized in that the compound represented by Formula 2 is phenolsulfonic acid or p-toluenesulfonic acid.

A cosmetic according to the present invention is characterized by including the layered double hydroxide according to any one of Claims 1 to 5.

### (Basic structure)

A layered double hydroxide according to the present invention has a structure including base layers each including a metal double hydroxide represented by the formula: M(II)_{1-X}M(III)_{X}(OH)₂ (wherein M(II) represents one or two bivalent metal atoms; M(III) represents a trivalent metal atom; and x represents 0.2 to 0.33), and a compound represented by a specific chemical formula (intermediate layer) and interlayer water each intercalated between the base layers. In this way, use of a compound represented by a specific chemical formula as an intercalation compound can provide a layered double hydroxide which has a neutral pH value upon being dispersed in water and has selective adsorbability to specific unsaturated fatty acids such as oleic acid.

In the present invention, the neutral pH value is a weakly acidic to weakly alkaline pH value, more specifically, a pH value in the range of 5 to 9. Incidentally, a layered double hydroxide according to the present invention can be adjusted so as to have a desired pH within the above-described range by adjusting a ratio of an anion to base layers described below. Incidentally, when a layered double hydroxide according to the present invention is added as a cosmetic, from the standpoint of, for example, influence on the skin, the layered double hydroxide preferably has, within the above-described pH range, a weakly acidic pH value of about 6 to a neutral pH value of about 7.

### (Bivalent metal atom)

The bivalent metal atom forming the base layers of a layered double hydroxide according to the present invention is not particularly limited. Various bivalent metal atoms such as Zn, Mg, Mn, Fe, Co, Ni, Cu, and Ca can be used. From the standpoint of, for example, stability, safety, and selective adsorbability of the layered double hydroxide, any one of Zn, Mg, and a mixture of Zn and Mg is preferably used.

### (Trivalent metal atom)

The trivalent metal atom forming the base layers of a layered double hydroxide according to the present invention is also not particularly limited. Various trivalent metal atoms such as Al, Cr, Fe, Co, In, and Mn can be used. From the standpoint of, for example, stability and ease of production of the layered double hydroxide, Al is preferably used.

### (Intercalation compound)

An anion forming the intermediate layer of a layered double hydroxide according to the present invention needs to be selected from compounds represented by the following chemical formulae.

[Formula 1] R₁-COOH

(wherein R₁ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group)

[Formula 2] R₂-SO₃H

(wherein R₂ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group).

Examples of the aliphatic hydrocarbon group include an alkyl group, an alkenyl group, an alkynyl group, and a cycloalkyl group.

Examples of the aromatic hydrocarbon group include a phenyl group, a naphthyl group, an anthryl group, and a phenanthryl group.

Examples of the heterocyclic group include a pyridyl group, a furanyl group, a pyranyl group, a thienyl group, a pyrrolidinyl group, an imidazolyl group, an imidazolinyl group, an imidazolidinyl group, a pyrazolyl group, a pyrazolinyl group, a pyrazolidinyl group, a pyridazinyl group, a pyrazinyl group, a piperidinyl group, a piperazinyl group, a thiolanyl group, a thianyl group, a quinolyl group, an isoquinolyl group, a benzofuranyl group, a benzothienyl group, an indolyl group, a carbazolyl group, a benzooxazolyl group, a benzothiazolyl group, a quinoxalyl group, a benzoimidazolyl group, a pyrazolyl group, a dibenzofuranyl group, a dibenzothienyl group, and a carbolinyl group.

Specific examples of Formula 1 wherein R₁ represents a substituted aliphatic hydrocarbon group include butanoic acid (butyric acid), pentanoic acid (valeric acid), hexanoic acid (caproic acid), heptanoic acid (enanthic acid), octanoic acid (caprylic acid), nonanoic acid (pelargonic acid), decanoic acid (capric acid), dodecanoic acid (lauric acid), tetradecanoic acid (myristic acid), pentadecanoic acid (pentadecylic acid), hexadecanoic acid (palmitic acid), heptadecanoic acid (margaric acid), octadecanoic acid (stearic acid), eicosanoic acid (arachidic acid), docosanoic acid (behenic acid), isobutyric acid, isopentanoic acid, pivalic acid, isohexanoic acid, isoheptanoic acid, isooctanoic acid, dimethyloctanoic acid, isononanoic acid, isodecanoic acid, isoundecanoic acid, isododecanoic acid, isotridecanoic acid, isotetradecanoic acid, isopentadecanoic acid, isohexadecanoic acid, isoheptadecanoic acid, isooctadecanoic acid, 4-aminobutyric acid, 6-aminohexanoic acid, tranexamic acid, and sodium N-lauroylsarcosinate.

Specific examples wherein R₁ represents a substituted aromatic hydrocarbon group include salicylic acid, benzoic acid, hydroxybenzoic acid, aminobenzoic acid, methoxybenzoic acid (anisic acid), and cinnamic acid.

Specific examples wherein R₁ represents a substituted heterocyclic group include picolinic acid and pyrrolidonecarboxylic acid (PCA).

Specific examples wherein R₂ represents a substituted aliphatic hydrocarbon group include taurine.

Specific examples wherein R₂ represents a substituted aromatic hydrocarbon group include phenolsulfonic acid and p-toluenesulfonic acid (PTS).

Specific examples wherein R₂ represents a substituted heterocyclic group include sodium
benzotriazolylbutylphenolsulfonate,
hydroxybenzophenonesulfonic acid, and
dihydroxydimethoxybenzophenone disulfonic acid.

Incidentally, the above-described compounds may be various isomers. Such compounds may be used alone or in combination. Such compounds may be various derivatives or metal salts such as sodium salts or zinc salts.

Of these, from the standpoint of achieving a neutral pH value (i.e., a weakly acidic to weakly alkaline pH value) upon being dispersed in water and selective adsorbability to unsaturated fatty acids, preferably used are salicylic acid, hydroxybenzoic acid, aminobenzoic acid, methoxybenzoic acid (anisic acid), pentanoic acid (valeric acid), dodecanoic acid (lauric acid), octadecanoic acid (stearic acid), docosanoic acid (behenic acid), isopentanoic acid, isododecanoic acid, isooctadecanoic acid, 4-aminobutyric acid, 6-aminohexanoic acid, tranexamic acid, picolinic acid, taurine, pyrrolidonecarboxylic acid (PCA), and sodium N-lauroylsarcosinate.

In addition, from the standpoint of achieving a weakly acidic pH value of about 6 to a neutral pH value of about 7 upon being dispersed in water, preferably used are benzoic acid, hydroxybenzoic acid, aminobenzoic acid, methoxybenzoic acid (anisic acid), pentanoic acid (valeric acid), dodecanoic acid (lauric acid), isooctadecanoic acid, tranexamic acid, pyrrolidonecarboxylic acid (PCA), sodium N-lauroylsarcosinate, phenolsulfonic acid, and p-toluenesulfonic acid (PTS).

### (Molar ratio of intercalation compound to base layers)

In a layered double hydroxide according to the present invention, a ratio of an intercalation compound to base layers is appropriately adjusted in accordance with, for example, the combination of bivalent and trivalent metal atoms and the intercalation compound and a target pH at the time of use as a cosmetic. In particular, from the standpoint of adsorbability to unsaturated fatty acids and pH upon being dispersed in water, the ratio preferably satisfies the intercalation compound/base layers = 0.05/1 to 5/1 (molar ratio), more preferably intercalation compound/base layers = 0.1/1 to 4/1 (molar ratio), still more preferably intercalation compound/base layers = 1/1 to 4/1 (molar ratio).

### (Production method)

As a method for producing a layered double hydroxide according to the present invention, known methods for producing layered double hydroxides can be used, such as the coprecipitation method, the ion exchange method, and the reconstruction method.

Specifically, the coprecipitation method for producing a layered double hydroxide is as follows: an aqueous solution mixture of bivalent and trivalent metal ions is added to an anion aqueous solution of the intercalation compound to cause hydrolysis for the metal ions, so that a metal double hydroxide forming base layers is formed and the intercalation compound is incorporated as an intermediate layer.

The ion exchange method is as follows: a layered double hydroxide in which anions having a low charge density are incorporated as an intermediate layer is produced in advance; and the layered double hydroxide is subsequently added to an anion aqueous solution of a desired intercalation compound to cause ion exchange from the earlier incorporated anions, to thereby produce a desired layered double hydroxide.

The reconstruction method is a method for producing a layered double hydroxide by the following procedures. First, what is called the carbonate layered double hydroxide (carbonate LDH) in which carbonate ions are incorporated as an intermediate layer is produced in advance. Subsequently, the carbonate LDH is fired (thermally decomposed) to cause decomposition of carbonate ions, emission of carbonic acid gas, release of interlayer water, and dehydration condensation of metal hydroxide forming base layers to produce a thermal decomposition product. Finally, this thermal decomposition product is added to or immersed in an anion aqueous solution of an intercalation compound; subsequently a process such as filtration or decantation is optionally used to remove excess anion component; and precipitate is collected. Thus, a desired layered double hydroxide is produced.

Of the above-described methods, the reconstruction method is preferably employed because of ease of synthesis, for example.

### (Cosmetic)

As described above, a layered double hydroxide according to the present invention has a neutral pH value (i.e., a weakly acidic to weakly alkaline pH value) upon being dispersed in water. Accordingly, the layered double hydroxide itself without being subjected to neutralization treatment or the like can be added as a raw material for a cosmetic. As a result, the cosmetic which has selective adsorbability to specific unsaturated fatty acids such as oleic acid can be produced.

The amount of a layered double hydroxide according to the present invention added for producing a cosmetic is not particularly limited and is appropriately determined in accordance with the need.

### Advantageous Effects of Invention

For a layered double hydroxide according to the present invention, benzoic acid or a derivative thereof is intercalated between two- or three-component base layers. This provides a layered double hydroxide which has a neutral pH value (i.e., a weakly acidic to weakly alkaline pH value) upon being dispersed in water and has selective adsorbability to specific unsaturated fatty acids such as oleic acid. Also, the layered double hydroxide obtained does not have an odor such as acetic acid odor.

A cosmetic according to the present invention, the cosmetic being produced using this layered double hydroxide, has selective adsorbability to unsaturated fatty acids. Accordingly, the cosmetic can effectively address makeup coming off and shine.

In particular, a layered double hydroxide according to the present invention employs specific metal ions and a specific compound for base layers and an intermediate layer. Accordingly, a layered double hydroxide which has higher selective adsorbability to unsaturated fatty acids can be obtained.

### Description of Embodiments

Hereinafter, a layered double hydroxide according to the present invention and a cosmetic produced using this layered double hydroxide will be described in detail with reference to examples. However, the present invention is not limited to the following examples.

### EXAMPLES

### (Example 1)

First, to a 1 mol/l Na₂CO₃ aqueous solution (2 1), a 1 mol/l ZnCl₂ aqueous solution (2.6 1) and a 1 mol/l AlCl₃ aqueous solution (1.4 1) were dropped while the pH of the reaction solution was maintained to be 7; and the solution was aged at 40°C for 1 hour. After that, the supernatant fluid of the mixture was removed; a 1 mol/l Na₂CO₃ aqueous solution (2 1) was subsequently added; and the mixture was heated at reflux for 5 hours. The resultant precipitate was collected, rinsed with water, and then vacuum-dried and pulverized at 60°C for 24 hours to provide a Zn-Al-based carbonate LDH.

Subsequently, this Zn-Al-based carbonate LDH was heated at 450°C for 20 hours to provide a thermal decomposition product.

Subsequently, 4.2 g of benzoic acid was added to 100 ml of water. Then, 1.37 g of sodium hydroxide was added and the solution was stirred to dissolve benzoic acid. To this aqueous solution, 11.8 g of the thermal decomposition product was added and the solution was stirred at room temperature for 15 hours to provide precipitate. In this case, the molar ratio (B/A) of benzoic acid (B) to base layers (A) was 1.25/1.

Finally, the precipitate was collected and then dried at 90°C for 20 hours and pulverized to provide a layered double hydroxide of Example 1 in which benzoic acid was intercalated.

### (Example 2)

A layered double hydroxide of Example 2 was obtained as in Example 1 except that the sodium hydroxide was changed to 2.34 g of 25% by weight of aqueous ammonia.

### (Example 3)

A layered double hydroxide of Example 3 was obtained as in Example 1 except that the amount of the thermal decomposition product was changed to 7.5 g. In this case, the molar ratio (B/A) of benzoic acid (B) to base layers (A) was 2/1.

### (Example 4)

A layered double hydroxide of Example 4 was obtained as in Example 1 except that the amount of the thermal decomposition product was changed to 7.5 g and the benzoic acid was changed to 4.8 g of salicylic acid. In this case, the molar ratio (B/A) of salicylic acid (B) to base layers (A) was 2/1.

### (Example 5)

A layered double hydroxide of Example 5 was obtained as in Example 1 except that the amount of the thermal decomposition product was changed to 7.5 g and the benzoic acid was changed to 5.2 g of p-anisic acid. In this case, the molar ratio (B/A) of p-anisic acid (B) to base layers (A) was 2/1.

### (Example 6)

A layered double hydroxide of Example 6 was obtained as in Example 5 except that the sodium hydroxide was changed to 2.34 g of 25% by weight of aqueous ammonia.

### (Example 7)

First, a Mg-Al-based carbonate LDH (DHT-6 manufactured by Kyowa Chemical Industry Co., Ltd.) was heated at 700°C for 20 hours to provide a thermal decomposition product.

Subsequently, 2.1 g of benzoic acid was added to 100 ml of water. Then, 0.69 g of sodium hydroxide was added and the solution was stirred to dissolve benzoic acid. To this aqueous solution, 60 g of the thermal decomposition product was added and the solution was stirred at room temperature for 15 hours to provide precipitate. In this case, the molar ratio (B/A) of benzoic acid (B) to base layers (A) was 0.1/1.

Finally, the precipitate was collected, subsequently dried at 90°C for 20 hours and pulverized to provide a layered double hydroxide of Example 7 in which benzoic acid was intercalated.

### (Example 8)

A layered double hydroxide of Example 8 was obtained as in Example 7 except that the amount of the thermal decomposition product was changed to 30 g, the amount of benzoic acid was changed to 5.2 g, and the amount of sodium hydroxide was changed to 1.73 g. In this case, the molar ratio (B/A) of benzoic acid (B) to base layers (A) was 0.5/1.

### (Example 9)

A layered double hydroxide of Example 9 was obtained as in Example 7 except that the amount of the thermal decomposition product was changed to 7.4 g, the amount of benzoic acid was changed to 5.2 g, and the amount of sodium hydroxide was changed to 1.73 g. In this case, the molar ratio (B/A) of benzoic acid (B) to base layers (A) was 2/1.

### (Example 10)

A layered double hydroxide of Example 10 was obtained as in Example 7 except that the amount of the thermal decomposition product was changed to 5.9 g, the benzoic acid was changed to 5.2 g of p-anisic acid, and the amount of sodium hydroxide was changed to 1.37 g. In this case, the molar ratio (B/A) of p-anisic acid (B) to base layers (A) was 2/1.

### (Example 11)

A layered double hydroxide of Example 11 was obtained as in Example 10 except that the sodium hydroxide was changed to 2.34 g of 25% by weight of aqueous ammonia.

### (Example 12)

First, a Zn-Mg-Al-based carbonate LDH (ALCAMIZER manufactured by Kyowa Chemical Industry Co., Ltd.) was heated at 600°C for 20 hours to provide a thermal decomposition product.

Subsequently, 4.2 g of benzoic acid was added to 100 ml of water. Then, 1.37 g of sodium hydroxide was added and the solution was stirred to dissolve benzoic acid. To this aqueous solution, 41.9 g of the thermal decomposition product was added and the solution was stirred at room temperature for 15 hours to provide precipitate. In this case, the molar ratio (B/A) of benzoic acid (B) to base layers (A) was 0.25/1.

Finally, the precipitate was collected, subsequently dried at 90°C for 20 hours and pulverized to provide a layered double hydroxide of Example 12 in which benzoic acid was intercalated.

### (Example 13)

A layered double hydroxide of Example 13 was obtained as in Example 12 except that the amount of the thermal decomposition product was changed to 8.4 g. In this case, the molar ratio (B/A) of benzoic acid (B) to base layers (A) was 1.25/1.

### (Example 14)

A layered double hydroxide of Example 14 was obtained as in Example 12 except that the amount of the thermal decomposition product was changed to 5.2 g. In this case, the molar ratio (B/A) of benzoic acid (B) to base layers (A) was 2/1.

### (Example 15)

A layered double hydroxide of Example 15 was obtained as in Example 14 except that the sodium hydroxide was changed to 2.34 g of 25% by weight of aqueous ammonia.

### (Example 16)

A layered double hydroxide of Example 16 was obtained as in Example 14 except that the benzoic acid was changed to 4.7 g of salicylic acid. In this case, the molar ratio (B/A) of salicylic acid (B) to base layers (A) was 2/1.

### (Example 17)

A layered double hydroxide of Example 17 was obtained as in Example 14 except that the benzoic acid was changed to 4.7 g of 3-hydroxybenzoic acid. In this case, the molar ratio (B/A) of 3-hydroxybenzoic acid (B) to base layers (A) was 2/1.

### (Example 18)

A layered double hydroxide of Example 18 was obtained as in Example 14 except that the benzoic acid was changed to 4.7 g of p-aminobenzoic acid. In this case, the molar ratio (B/A) of 3-hydroxybenzoic acid (B) to base layers (A) was 2/1.

### (Example 19)

A layered double hydroxide of Example 19 was obtained as in Example 12 except that the amount of the thermal decomposition product was changed to 5.3 g, the benzoic acid was changed to 1.6 g of p-anisic acid, and the amount of sodium hydroxide was changed to 0.42 g. In this case, the molar ratio (B/A) of p-anisic acid (B) to base layers (A) was 0.6/1.

### (Example 20)

A layered double hydroxide of Example 20 was obtained as in Example 19 except that the amount of p-anisic acid was changed to 2.6 g and the amount of sodium hydroxide was changed to 0.68 g. In this case, the molar ratio (B/A) of p-anisic acid (B) to base layers (A) was 1/1.

### (Example 21)

A layered double hydroxide of Example 21 was obtained as in Example 19 except that the amount of p-anisic acid was changed to 3.8 g and the amount of sodium hydroxide was changed to 1 g. In this case, the molar ratio (B/A) of p-anisic acid (B) to base layers (A) was 1.45/1.

### (Example 22)

A layered double hydroxide of Example 22 was obtained as in Example 19 except that the amount of p-anisic acid was changed to 4.7 g and the amount of sodium hydroxide was changed to 1.24 g. In this case, the molar ratio (B/A) of p-anisic acid (B) to base layers (A) was 1.8/1.

### (Example 23)

A layered double hydroxide of Example 23 was obtained as in Example 22 except that the sodium hydroxide was changed to 2.1 g of 25% by weight of aqueous ammonia. In this case, the molar ratio (B/A) of p-anisic acid (B) to base layers (A) was 1.8/1.

### (Example 24)

A layered double hydroxide of Example 24 was obtained as in Example 23 except that the amount of p-anisic acid was changed to 5.2 g and the amount of 25% by weight of aqueous ammonia was changed to 2.34 g. In this case, the molar ratio (B/A) of p-anisic acid (B) to base layers (A) was 2/1.

### (Example 25)

A layered double hydroxide of Example 25 was obtained as in Example 23 except that the amount of p-anisic acid was changed to 5.7 g and the amount of 25% by weight of aqueous ammonia was changed to 2.57 g. In this case, the molar ratio (B/A) of p-anisic acid (B) to base layers (A) was 2.2/1.

### (Example 26)

First, a Zn-Al-based carbonate LDH was produced as in Example 1.

Subsequently, this Zn-Al-based carbonate LDH was heated at 450°C for 20 hours to provide a thermal decomposition product.

Subsequently, 6 g of 3-hydroxybenzoic acid was added to 100 ml of water. Then, 1.73 g of sodium hydroxide was added and the solution was stirred to dissolve 3-hydroxybenzoic acid. To this aqueous solution, 6 g of the thermal decomposition product was added and the solution was stirred at room temperature for 15 hours to provide precipitate. In this case, the molar ratio (B/A) of 3-hydroxybenzoic acid (B) to base layers (A) was 2/1.

Finally, the precipitate was collected, subsequently dried at 90°C for 20 hours and pulverized to provide a layered double hydroxide of Example 26 in which 3-hydroxybenzoic acid was intercalated.

### (Example 27)

A layered double hydroxide of Example 27 was obtained as in Example 26 except that the 3-hydroxybenzoic acid was changed to 6 g of p-aminobenzoic acid. In this case, the molar ratio (B/A) of p-aminobenzoic acid (B) to base layers (A) was 2/1.

### (Example 28)

A layered double hydroxide of Example 28 was obtained as in Example 26 except that the 3-hydroxybenzoic acid was changed to 6 g of pentanoic acid. In this case, the molar ratio (B/A) of pentanoic acid (B) to base layers (A) was 4/1.

### (Example 29)

A layered double hydroxide of Example 29 was obtained as in Example 26 except that the 3-hydroxybenzoic acid was changed to 6 g of dodecanoic acid. In this case, the molar ratio (B/A) of dodecanoic acid (B) to base layers (A) was 2/1.

### (Example 30)

A layered double hydroxide of Example 30 was obtained as in Example 26 except that the 3-hydroxybenzoic acid was changed to 3 g of dodecanoic acid. In this case, the molar ratio (B/A) of dodecanoic acid (B) to base layers (A) was 1/1.

### (Example 31)

A layered double hydroxide of Example 31 was obtained as in Example 26 except that the 3-hydroxybenzoic acid was changed to 6 g of tetradecanoic acid. In this case, the molar ratio (B/A) of tetradecanoic acid (B) to base layers (A) was 1/1.

### (Example 32)

A layered double hydroxide of Example 32 was obtained as in Example 26 except that the 3-hydroxybenzoic acid was changed to 6 g of hexadecanoic acid. In this case, the molar ratio (B/A) of hexadecanoic acid (B) to base layers (A) was 1/1.

### (Example 33)

A layered double hydroxide of Example 33 was obtained as in Example 26 except that the 3-hydroxybenzoic acid was changed to 6 g of octadecanoic acid. In this case, the molar ratio (B/A) of octadecanoic acid (B) to base layers (A) was 2/1.

### (Example 34)

A layered double hydroxide of Example 34 was obtained as in Example 26 except that the 3-hydroxybenzoic acid was changed to 3 g of octadecanoic acid. In this case, the molar ratio (B/A) of octadecanoic acid (B) to base layers (A) was 1/1.

### (Example 35)

A layered double hydroxide of Example 35 was obtained as in Example 26 except that the 3-hydroxybenzoic acid was changed to 6 g of docosanoic acid. In this case, the molar ratio (B/A) of docosanoic acid (B) to base layers (A) was 1/1.

### (Example 36)

A layered double hydroxide of Example 36 was obtained as in Example 26 except that the 3-hydroxybenzoic acid was changed to 6 g of isooctadecanoic acid. In this case, the molar ratio (B/A) of isooctadecanoic acid (B) to base layers (A) was 2/1.

### (Example 37)

A layered double hydroxide of Example 37 was obtained as in Example 26 except that the 3-hydroxybenzoic acid was changed to 3 g of isooctadecanoic acid. In this case, the molar ratio (B/A) of isooctadecanoic acid (B) to base layers (A) was 1/1.

### (Example 38)

A layered double hydroxide of Example 38 was obtained as in Example 26 except that the 3-hydroxybenzoic acid was changed to 6 g of 4-aminobutyric acid. In this case, the molar ratio (B/A) of 4-aminobutyric acid (B) to base layers (A) was 4/1.

### (Example 39)

A layered double hydroxide of Example 39 was obtained as in Example 26 except that the 3-hydroxybenzoic acid was changed to 6 g of 6-aminohexanoic acid. In this case, the molar ratio (B/A) of 6-aminohexanoic acid (B) to base layers (A) was 3.3/1.

### (Example 40)

A layered double hydroxide of Example 40 was obtained as in Example 26 except that the 3-hydroxybenzoic acid was changed to 6 g of tranexamic acid. In this case, the molar ratio (B/A) of tranexamic acid (B) to base layers (A) was 2.7/1.

### (Example 41)

A layered double hydroxide of Example 41 was obtained as in Example 26 except that the 3-hydroxybenzoic acid was changed to 6 g of picolinic acid. In this case, the molar ratio (B/A) of picolinic acid (B) to base layers (A) was 3.5/1.

### (Example 42)

A layered double hydroxide of Example 42 was obtained as in Example 26 except that the 3-hydroxybenzoic acid was changed to 6 g of taurine. In this case, the molar ratio (B/A) of taurine (B) to base layers (A) was 3.5/1.

### (Example 43)

A layered double hydroxide of Example 43 was obtained as in Example 26 except that the 3-hydroxybenzoic acid was changed to 6 g of pyrrolidonecarboxylic acid. In this case, the molar ratio (B/A) of pyrrolidonecarboxylic acid (B) to base layers (A) was 3.3/1.

### (Example 44)

A layered double hydroxide of Example 44 was obtained as in Example 26 except that the 3-hydroxybenzoic acid was changed to 6 g of sodium N-lauroylsarcosinate. In this case, the molar ratio (B/A) of sodium N-lauroylsarcosinate (B) to base layers (A) was 1.5/1.

### (Example 45)

A layered double hydroxide of Example 45 was obtained as in Example 26 except that the 3-hydroxybenzoic acid was changed to 6 g of phenolsulfonic acid. In this case, the molar ratio (B/A) of phenolsulfonic acid (B) to base layers (A) was 2/1.

### (Example 46)

A layered double hydroxide of Example 46 was obtained as in Example 26 except that the 3-hydroxybenzoic was changed to 6 g of p-toluenesulfonic acid. In this case, the molar ratio (B/A) of p-toluenesulfonic acid (B) to base layers (A) was 2.5/1.

### (Comparative example 1)

A layered double hydroxide of Comparative example 1 was obtained as in Example 7 except that the amount of the thermal decomposition product was changed to 10.1 g and the benzoic acid was changed to 6.3 g of magnesium acetate tetrahydrate, which was dissolved in water without use of sodium hydroxide. In this case, the molar ratio (B/A) of magnesium acetate (B) to base layers (A) was 1/1.

### (Comparative example 2)

A layered double hydroxide of Comparative example 2 was obtained as in Example 12 except that the amount of the thermal decomposition product was changed to 5.3 g and the benzoic acid was changed to 5.9 g of toluenesulfonic acid. In this case, the molar ratio (B/A) of toluenesulfonic acid (B) to base layers (A) was 2/1.

### (Comparative example 3)

The thermal decomposition product of the Zn-Mg-Al-based carbonate LDH to be subjected to the intercalation treatment (the thermal decomposition product used for the layered double hydroxide of Example 12) was defined as a layered double hydroxide of Comparative example 3.

### (Comparative example 4)

The Zn-Mg-Al-based carbonate LDH to be subjected to the thermal decomposition product was defined as a layered double hydroxide of Comparative example 4.

Subsequently, Examples and Comparative examples were subjected to pH measurement, odor evaluation, water repellency evaluation, and evaluation of adsorbability to unsaturated fatty acids.

### (pH measurement)

The pH measurement for each of the layered double hydroxides of Examples and Comparative examples was performed by measuring the pH of a dispersion of 1% by weight hydroxide in pure water. The results are described in Table 1 and Table 2.

### (Odor evaluation)

The odor evaluation was performed by using a sensory evaluation in which five male and five female evaluators (in total ten evaluators) evaluate the odor of each of the layered double hydroxides of Examples and Comparative examples. The results are described in Table 1 and Table 2.

### (Water repellency evaluation)

The water repellency evaluation was performed by using the following procedures. The results are described in Table 1 and Table 2.
1) Place 50 ml of water into a graduated cylinder.
2) Place 1.0 g of a layered double hydroxide into the graduated cylinder prepared in 1).
3) Perform agitation for the graduated cylinder prepared in 2) by turning the cylinder upside down ten times with the opening being closed by hand.
4) After the agitation, leave the graduated cylinder at rest and determine by observation as to whether the layered double hydroxide floats on the water surface without being dispersed in water (whether the layered double hydroxide has water repellency) or not.

### (Evaluation of adsorbability to unsaturated fatty acids)

The evaluation for adsorbability to unsaturated fatty acids (selective adsorbability to sebum components) was performed in the following manner.

First, 0.5 g of each of the layered double hydroxides of Examples and Comparative examples was weighed and placed into 10 ml sample tubes. To these tubes, 1.5 g of fats and oils were added and then stirred with a pencil mixer for 5 minutes to prepare samples.

Subsequently, the fluidity of each sample after the lapse of 5 minutes was evaluated in accordance with the following evaluation system. The fats and oils were oleic acid, triolein, squalene, glyceryl tri(caprylate/caprate), glyceryl tri(2-ethylhexanoate), and liquid paraffin. The results are described in Table 1 and Table 2.

### [Evaluation system]

A: after the lapse of 2 minutes, no fluidity is observed and solidification is observed
B: no fluidity is observed and solidification is observed
C: fluidity and thickening are observed
D: fluidity and no thickening are observed

**[Table 1]**

| | Base layers (A) | Intercalation compound (B) | Molar ratio (B/A) | Evaluation items | | | | | | | | |
|---|---|---|---|---|---|---|---|---|---|---|---|---|
| | | | | pH of 1 wt% aqueous dispersion | Acetic acid odor | Water repellency | Evaluation for adsorbability to unsaturated fatty acids | | | | | |
| | | | | | | | Oleic acid | Triolein | Squalene | Glyceryl tri(caprylate/caprate) | Glyceryl tri(2-ethylhexanoate) | Liquid paraffin |
| Example 1 | Zn-Al | benzoic acid | 1.25/1 | 8.5 | Absent | Absent | B | D | D | D | D | D |
| Example 2 | | | 1.25/1 | 6.1 | Absent | Absent | B | D | D | D | D | D |
| Example 3 | | | 2.0/1 | 8.3 | Absent | Absent | B | D | D | D | D | D |
| Example 4 | | salicylic acid | 2.0/1 | 8.5 | Absent | Absent | B | D | D | D | D | D |
| Example 5 | | p-anisic acid | 2.0/1 | 8.4 | Absent | Absent | B | D | D | D | D | D |
| Example 6 | | | 2.0/1 | 6.1 | Absent | Absent | A | D | D | D | D | D |
| Example 7 | Mg-Al | benzoic acid | 0.1/1 | 8.5 | Absent | Absent | B | D | D | D | D | D |
| Example 8 | | | 0.5/1 | 8.4 | Absent | Absent | B | D | D | D | D | D |
| Example 9 | | | 2.0/1 | 8.4 | Absent | Absent | B | D | D | D | D | D |
| Example 10 | | p-anisic acid | 2.0/1 | 8.5 | Absent | Absent | B | D | D | D | D | D |
| Example 11 | | | 2.0/1 | 8.3 | Absent | Absent | B | D | D | D | D | D |
| Example 12 | | benzoic acid | 0.25/1 | 8.4 | Absent | Absent | B | D | D | D | D | D |
| Example 13 | | | 1.25/1 | 8.3 | Absent | Absent | B | D | D | D | D | D |
| Example 14 | | | 2.0/1 | 8.3 | Absent | Absent | B | D | D | D | D | D |
| Example 15 | | | 2.0/1 | 6.0 | Absent | Absent | B | D | D | D | D | D |
| Example 16 | | salicylic acid | 2.0/1 | 8.3 | Absent | Absent | B | D | D | D | D | D |
| Example 17 | | 3-hydroxybenzoic acid | 2.0/1 | 8.4 | Absent | Absent | B | D | D | D | D | D |
| Example 18 | Mg-Zn-Al | p-aminobenzoic acid | 2.0/1 | 8.5 | Absent | Absent | B | D | D | D | D | D |
| Example 19 | | p-anisic acid | 0.6/1 | 8.6 | Absent | Absent | B | D | D | D | D | D |
| Example 20 | | | 1/1 | 8.4 | Absent | Absent | B | D | D | D | D | D |
| Example 21 | | | 1.45/1 | 8.5 | Absent | Absent | B | D | D | D | D | D |
| Example 22 | | | 1.8/1 | 8.3 | Absent | Absent | B | D | D | D | D | D |
| Example 23 | | | 1.8/1 | 6.2 | Absent | Absent | B | D | D | D | D | D |
| Example 24 | | | 2/1 | 6.2 | Absent | Absent | B | D | D | D | D | D |
| Example 25 | | | 2.2/1 | 6.3 | Absent | Absent | B | D | D | D | D | D |
| Comparative example 1 | Mg-Al | Mg acetate·4H₂O | 1/1 | 10.0 | Acetic acid odor | Absent | B | D | D | D | D | D |
| Comparative example 2 | Mg-Zn-Al | toluenesulfonic acid | 2.0/1 | 8.4 | Absent | Absent | C | C | C | C | C | C |
| Comparative example 3 | Mg-Zn-Al | None | - | 10.0 | Absent | Absent | D | D | D | D | D | D |
| Comparative example 4 | Mg-Zn-Al | carbonate ion | - | 10.0 | Absent | Absent | D | D | D | D | D | D |

**[Table 2]**

| | Base layers (A) | Intercalation compound (B) | Molar ratio (B/A) | Evaluation items | | | | | | | | |
|---|---|---|---|---|---|---|---|---|---|---|---|---|
| | | | | pH of 1 wt% aqueous dispersion | Acetic acid odor | Water repellency | Evaluation for adsorbability to unsaturated fatty acids | | | | | |
| | | | | | | | Oleic acid | Triolein | Squalene | Glyceryl tri(caprylatelcaprate) | Glyceryl tri(2-ethylhexanoate) | Liquid paraffin |
| Example 26 | | 3-hydroxybenzoic acid | 2.0/1 | 7.5 | Absent | Absent | B | D | D | D | D | D |
| Example 27 | | p-aminobenzoic acid | 2.0/1 | 7.5 | Absent | Absent | B | D | D | D | D | D |
| Example 28 | | pentanoic acid | 4.0/1 | 7.2 | Absent | Present | B | D | D | D | D | D |
| Example 29 | | dodecanoic acid | 2.0/1 | 7.2 | Absent | Present | A | D | D | D | D | D |
| Example 30 | | | 1.0/1 | 6.8 | Absent | Present | A | D | D | D | D | D |
| Example 31 | | tetradecanoic acid | 1.0/1 | 7.5 | Absent | Present | A | D | D | D | D | D |
| Example 32 | | hexadecanoic acid | 1.0/1 | 8.0 | Absent | Present | A | D | D | D | D | D |
| Example 33 | | octadecanoic acid | 2.0/1 | 8.0 | Absent | Present | A | D | D | D | D | D |
| Example 34 | | | 1.0/1 | 8.0 | Absent | Present | A | D | D | D | D | D |
| Example 35 | | docosanoic acid | 1.0/1 | 8.0 | Absent | Present | B | D | D | D | D | D |
| Example 36 | | isooctadecanoic acid | 2.0/1 | 7.2 | Absent | Present | B | D | D | D | D | D |
| Example 37 | Zn-Al | | 1.0/1 | 7.5 | Absent | Present | B | D | D | D | D | D |
| Example 38 | | 4-aminobutyric acid | 4.0/1 | 8.5 | Absent | Absent | B | D | D | D | D | D |
| Example 39 | | 6-aminohexanoic acid | 3.3/1 | 8.9 | Absent | Absent | B | D | D | D | D | D |
| Example 40 | | tranexamic acid | 2.7/1 | 7.2 | Absent | Absent | B | D | D | D | D | D |
| Example 41 | | picolinic acid | 3.5/1 | 7.8 | Absent | Absent | B | D | D | D | D | D |
| Example 42 | | taurine | 3.5/1 | 7.8 | Absent | Absent | B | D | D | D | D | D |
| Example 43 | | pyrrolidonecarboxylic acid | 3.3/1 | 6.5 | Absent | Absent | B | D | D | D | D | D |
| Example 44 | | Na N-lauroylsarcosinate | 1.5/1 | 7.5 | Absent | Present | B | D | D | D | D | D |
| Example 45 | | phenolsulfonic acid | 2.0/1 | 7.5 | Absent | Absent | B | D | D | D | D | D |
| Example 46 | | p-toluenesulfonic acid | 2.5/1 | 7.0 | Absent | Absent | A | D | D | D | D | D |

The results in Table 1 and Table 2 indicate that all the layered double hydroxides of Examples in which specific compounds are intercalated have neutral pH values of 5 to 9 upon being dispersed in water, no odor, and selective adsorbability to unsaturated fatty acids (in particular, oleic acid). In particular, the layered double hydroxides of Examples 2, 6, 15, 23 to 31, 36, 37, 40, and 43 to 46 have no odor, selective adsorbability to unsaturated fatty acids (in particular, oleic acid), and a weakly acidic pH value of about 6 to a neutral pH value of about 7 upon being dispersed in water, so that these layered double hydroxides have been found to be ideal layered double hydroxides. In particular, of these, the layered double hydroxides of Examples 6, 29 to 34, and 46 have been found to have a higher selective adsorbability to unsaturated fatty acids (in particular, oleic acid) than the layered double hydroxides of the other Examples.

In Examples, in particular, the layered double hydroxides of Examples 28 to 37 and 44 have been found to have higher water repellency than the layered double hydroxides of the other Examples. Accordingly, use of layered double hydroxides of Examples 28 to 37 and 44 for cosmetics can impart, in addition to selective adsorbability to unsaturated fatty acids (in particular, oleic acid), water repellency to the cosmetics. As a result, makeup coming off and shine can be more effectively prevented.

In contrast, the layered double hydroxide of Comparative example 2 has been found to have a neutral pH value of 5 to 9 upon being dispersed in water and no odor, but found to have nonspecific adsorbability to unsaturated fatty acids and beneficial components such as squalene. The layered double hydroxide of Comparative example 3 has been found to have selective adsorbability to unsaturated fatty acids (in particular, oleic acid), but found to have a strongly alkaline pH value of 10 upon being dispersed in water as described in paragraphs [0007] and [0008], and also found to have acetic acid odor. The layered double hydroxides of Comparative examples 3 and 4 in which the specific compounds were not intercalated exhibited no adsorbability.

### (Evaluation for cosmetic)

Subsequently, the layered double hydroxides of Examples and Comparative examples 2 to 4 were used to produce powder foundations. The powder foundations were evaluated by a sensory evaluation for the presence or absence of shine on the skin to which the powder foundations were applied, for five male and five female evaluators (in total ten evaluators). Specifically, the skin of each evaluator was evaluated in terms of shine by using a point system (1 point for an evaluator having no shine and 0 point for an evaluator having shine). The powder foundations were produced in the following manner: a powder serving as component A and a liquid serving as component B were separately prepared in accordance with the formulations in Table 3; and the liquid of component B was then gradually added to the powder of component A. The results are described in Table 4 and Table 5. Incidentally, Comparative example 1 was not subjected to this evaluation because of strong acetic acid odor.

**[Table 3]**

| | Trade names | Cosmetic labeling names | Mixing ratio (% by weight) |
|---|---|---|---|
| | TAROX LLXLO (manufactured by Titan Kogyo, Ltd.) | yellow iron oxide | 1.41 |
| | TAROX R516-L (manufactured by Titan Kogyo, Ltd.) | red iron oxide | 0.35 |
| | TAROX R110-7 (manufactured by Titan Kogyo, Ltd.) | red iron oxide | 0.53 |
| | TAROX BL-100 (manufactured by Titan Kogyo, Ltd.) | black iron oxide | 0.18 |
| Component A | Mica R-1000 (manufactured by Kakuhachi Co., Ltd.) | mica | 42.53 |
| | JA-80R (manufactured by ASADA MILLING CO., LTD.) | talc | 20.00 |
| | Nylon 12 (manufactured by Torav Industries, Inc.) | nylon 6 | 5.00 |
| | MPY-1133 (manufactured by Tayca Corporation) | titanium oxide | 8.00 |
| | Layered double hydroxide of Example or Comparative example | | 10.00 |
| Component B | Crodalan SWL (manufactured by Croda Japan KK) | purified lanoline | 2.40 |
| | Squalane (manufactured by Nikko Chemicals Co., Ltd.) | squalane | 2.40 |
| | COCONARD MT (manufactured by Kao Corporation) | glyceryl tri(caprylate/caprate) | 1.80 |
| | TIO (manufactured by The Nisshin OilliO Group, Ltd.) | trioctanoin | 1.80 |
| | KF56 (manufactured by Shin-Etsu Chemical Co., Ltd.) | diphenylsiloxy phenyl trimethicone | 3.60 |
| | | | 100.00 |

**[Table 4]**

| | Layered double hydroxide used | Evaluators | | | | | | | | | | Total score |
|---|---|---|---|---|---|---|---|---|---|---|---|---|
| | | Males | | | | | Females | | | | | |
| | | 1 | 2 | 3 | 4 | 5 | 1 | 2 | 3 | 4 | 5 | |
| Example 47 | Layered double hydroxide of Example 1 | 1 | 1 | 1 | 1 | 1 | 1 | 1 | 1 | 1 | 1 | 10 |
| Example 48 | Layered double hydroxide of Example 2 | 1 | 1 | 0 | 1 | 1 | 1 | 1 | 1 | 1 | 1 | 9 |
| Example 49 | Layered double hydroxide of Example 3 | 1 | 1 | 1 | 1 | 1 | 1 | 1 | 1 | 1 | 1 | 10 |
| Example 50 | Layered double hydroxide of Example 4 | 1 | 1 | 1 | 0 | 1 | 1 | 1 | 1 | 1 | 1 | 9 |
| Example 51 | Layered double hydroxide of Example 5 | 1 | 1 | 1 | 1 | 1 | 1 | 1 | 1 | 1 | 1 | 10 |
| Example 52 | Layered double hydroxide of Example 6 | 1 | 1 | 1 | 1 | 1 | 1 | 1 | 1 | 1 | 1 | 10 |
| Example 53 | Layered double hydroxide of Example 7 | 1 | 1 | 1 | 1 | 1 | 1 | 1 | 1 | 0 | 1 | 9 |
| Example 54 | Layered double hydroxide of Example 8 | 1 | 1 | 0 | 1 | 1 | 1 | 1 | 1 | 1 | 1 | 9 |
| Example 55 | Layered double hydroxide of Example 9 | 1 | 1 | 1 | 1 | 1 | 1 | 1 | 1 | 1 | 1 | 10 |
| Example 56 | Layered double hydroxide of Example 10 | 1 | 1 | 1 | 1 | 1 | 1 | 1 | 1 | 1 | 1 | 10 |
| Example 57 | Layered double hydroxide of Example 11 | 1 | 1 | 0 | 1 | 1 | 1 | 1 | 1 | 1 | 1 | 9 |
| Example 58 | Layered double hydroxide of Example 12 | 1 | 1 | 1 | 1 | 1 | 0 | 1 | 1 | 1 | 1 | 9 |
| Example 59 | Layered double hydroxide of Example 13 | 1 | 1 | 1 | 1 | 1 | 1 | 0 | 1 | 1 | 1 | 9 |
| Example 60 | Layered double hydroxide of Example 14 | 1 | 1 | 1 | 1 | 1 | 1 | 1 | 1 | 1 | 1 | 10 |
| Example 61 | Layered double hydroxide of Example 15 | 1 | 1 | 1 | 1 | 1 | 1 | 1 | 1 | 1 | 1 | 10 |
| Example 62 | Layered double hydroxide of Example 16 | 1 | 1 | 1 | 1 | 1 | 1 | 1 | 1 | 1 | 1 | 10 |
| Example 63 | Layered double hydroxide of Example 17 | 1 | 1 | 1 | 1 | 1 | 1 | 1 | 1 | 1 | 1 | 10 |
| Example 64 | Layered double hydroxide of Example 18 | 1 | 1 | 0 | 1 | 1 | 1 | 1 | 1 | 1 | 1 | 9 |
| Example 65 | Layered double hydroxide of Example 19 | 1 | 1 | 1 | 1 | 1 | 1 | 1 | 1 | 1 | 0 | 9 |
| Example 66 | Layered double hydroxide of Example 20 | 0 | 1 | 1 | 1 | 1 | 1 | 1 | 1 | 1 | 1 | 9 |
| Example 67 | Layered double hydroxide of Example 21 | 1 | 1 | 1 | 1 | 1 | 1 | 1 | 1 | 1 | 1 | 10 |
| Example 68 | Layered double hydroxide of Example 22 | 1 | 1 | 1 | 1 | 1 | 1 | 1 | 1 | 1 | 1 | 10 |
| Example 69 | Layered double hydroxide of Example 23 | 1 | 1 | 1 | 1 | 1 | 1 | 1 | 1 | 1 | 1 | 10 |
| Example 70 | Layered double hydroxide of Example 24 | 1 | 1 | 1 | 1 | 1 | 1 | 1 | 1 | 0 | 1 | 9 |
| Example 71 | Layered double hydroxide of Example 25 | 1 | 1 | 1 | 1 | 1 | 1 | 1 | 1 | 1 | 1 | 10 |
| Comparative example 5 | Layered double hydroxide of Comparative example 2 | 0 | 1 | 0 | 0 | 0 | 0 | 0 | 1 | 0 | 1 | 3 |
| Comparative example 6 | Layered double hydroxide of Comparative example 3 | 0 | 0 | 0 | 0 | 0 | 0 | 0 | 0 | 0 | 0 | 0 |
| Comparative example 7 | Layered double hydroxide of Comparative example 4 | 0 | 0 | 0 | 0 | 0 | 0 | 0 | 0 | 0 | 0 | 0 |

**[Table 5]**

| | Layered double hydroxide used | Evaluators | | | | | | | | | | Total score |
|---|---|---|---|---|---|---|---|---|---|---|---|---|
| | | Males | | | | | Females | | | | | |
| | | 1 | 2 | 3 | 4 | 5 | 1 | 2 | 3 | 4 | 5 | |
| Example 72 | Layered double hydroxide of Example 26 | 1 | 1 | 1 | 1 | 0 | 1 | 1 | 1 | 1 | 1 | 9 |
| Example 73 | Layered double hydroxide of Example 27 | 1 | 0 | 1 | 1 | 1 | 1 | 1 | 1 | 1 | 1 | 9 |
| Example 74 | Layered double hydroxide of Example 28 | 1 | 0 | 1 | 1 | 1 | 1 | 1 | 1 | 1 | 1 | 9 |
| Example 75 | Layered double hydroxide of Example 29 | 1 | 1 | 1 | 1 | 1 | 1 | 1 | 1 | 1 | 1 | 10 |
| Example 76 | Layered double hydroxide of Example 30 | 1 | 1 | 1 | 1 | 1 | 1 | 1 | 1 | 1 | 1 | 10 |
| Example 77 | Layered double hydroxide of Example 31 | 1 | 1 | 1 | 1 | 1 | 1 | 1 | 1 | 1 | 1 | 10 |
| Example 78 | Layered double hydroxide of Example 32 | 1 | 1 | 1 | 1 | 1 | 1 | 1 | 1 | 1 | 1 | 10 |
| Example 79 | Layered double hydroxide of Example 33 | 1 | 1 | 1 | 1 | 1 | 1 | 1 | 1 | 1 | 1 | 10 |
| Example 80 | Layered double hydroxide of Example 34 | 1 | 1 | 1 | 1 | 1 | 1 | 1 | 1 | 1 | 1 | 10 |
| Example 81 | Layered double hydroxide of Example 35 | 1 | 1 | 1 | 0 | 1 | 1 | 1 | 1 | 1 | 1 | 9 |
| Example 82 | Layered double hydroxide of Example 36 | 1 | 1 | 1 | 1 | 1 | 1 | 1 | 1 | 1 | 0 | 9 |
| Example 83 | Layered double hydroxide of Example 37 | 1 | 1 | 1 | 1 | 1 | 1 | 1 | 0 | 1 | 1 | 9 |
| Example 84 | Layered double hydroxide of Example 38 | 1 | 1 | 1 | 1 | 0 | 1 | 1 | 1 | 1 | 1 | 9 |
| Example 85 | Layered double hydroxide of Example 39 | 0 | 1 | 1 | 1 | 1 | 1 | 1 | 1 | 1 | 1 | 9 |
| Example 86 | Layered double hydroxide of Example 40 | 1 | 1 | 0 | 1 | 1 | 1 | 1 | 1 | 1 | 1 | 9 |
| Example 87 | Layered double hydroxide of Example 41 | 1 | 1 | 1 | 0 | 1 | 1 | 1 | 1 | 1 | 1 | 9 |
| Example 88 | Layered double hydroxide of Example 42 | 0 | 1 | 1 | 1 | 1 | 1 | 1 | 1 | 1 | 1 | 9 |
| Example 89 | Layered double hydroxide of Example 43 | 1 | 1 | 1 | 1 | 1 | 0 | 1 | 1 | 1 | 1 | 9 |
| Example 90 | Layered double hydroxide of Example 44 | 1 | 1 | 1 | 1 | 0 | 1 | 1 | 1 | 1 | 1 | 9 |
| Example 91 | Layered double hydroxide of Example 45 | 1 | 1 | 1 | 0 | 1 | 1 | 1 | 1 | 1 | 1 | 9 |
| Example 92 | Layered double hydroxide of Example 46 | 1 | 1 | 1 | 1 | 1 | 1 | 1 | 1 | 1 | 1 | 10 |

The results in Table 4 and Table 5 indicate that the cosmetics of Examples 47 to 92 produced using the layered double hydroxides of Examples 1 to 46 do not cause shine in most of the evaluators and selective adsorbability to unsaturated fatty acids (in particular, oleic acid) is also provided in the form of cosmetics.

In contrast, the layered double hydroxides of Comparative examples 2 to 4 have low adsorbability to unsaturated fatty acids (oleic acid) and, as a result, cannot effectively prevent occurrence of shine in the form of cosmetics (Comparative examples 5 to 7).

As described above, regarding a layered double hydroxide according to the present invention and a cosmetic produced using this layered double hydroxide, a layered double hydroxide can be provided which has a neutral pH value (i.e., a weakly acidic to weakly alkaline pH value) upon being dispersed in water, has selective adsorbability to specific unsaturated fatty acids such as oleic acid, and does not have odor such as acetic acid odor.

### Industrial Applicability

A layered double hydroxide according to the present invention can be used for producing a cosmetic and, in particular, can be used for selective adsorption to unsaturated fatty acids such as oleic acid.

## Claims

1. A layered double hydroxide which has selective adsorbability to unsaturated fatty acids, **characterized by** comprising:
base layers each comprising a metal double hydroxide represented by the formula:
M(II)_{1-X}M(III)_{X}(OH)₂
(wherein M(II) represents one or two bivalent metal atoms; M(III) represents a trivalent metal atom; and x represents 0.2 to 0.33); and
an intermediate layer and interlayer water each intercalated between the base layers,
wherein the intermediate layer comprises
a compound represented by the following Formula 1 or Formula 2
[Formula 1] R₁-COOH
(wherein R₁ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group)
[Formula 2] R₂-SO₃H
(wherein R₂ represents at least one substituent selected from an aliphatic hydrocarbon group, a substituted aliphatic hydrocarbon group, an aromatic hydrocarbon group, a substituted aromatic hydrocarbon group, a heterocyclic group and a substituted heterocyclic group).

2. The layered double hydroxide according to Claim 1, **characterized in that**
the M(II) represents Zn, and
the M(III) represents Al.

3. The layered double hydroxide according to Claim 1, **characterized in that**
the M(II) represents Mg and Zn, and
the M(III) represents Al.

4. The layered double hydroxide according to any one of Claims 1 to 3, **characterized in that**
the compound represented by the Formula 1 is
at least one compound selected from salicylic acid, hydroxybenzoic acid, aminobenzoic acid, methoxybenzoic acid, pentanoic acid, dodecanoic acid, octadecanoic acid, docosanoic acid, isopentanoic acid, isododecanoic acid, isooctadecanoic acid, 4-aminobutyric acid, 6-aminohexanoic acid, tranexamic acid, picolinic acid, taurine, pyrrolidonecarboxylic acid, and sodium N-lauroylsarcosinate.

5. The layered double hydroxide according to any one of Claims 1 to 3, **characterized in that**
the compound represented by the Formula 2 is phenolsulfonic acid or p-toluenesulfonic acid.

6. A cosmetic **characterized by** comprising the layered double hydroxide according to any one of Claims 1 to 5.
